# EUROPEAN PATENT SPECIFICATION

(11) **EP 2 226 014 B1**
(45) Date of publication and mention of the grant of the patent: **14.12.2011**
(21) Application number: 09305209.0
(22) Date of filing: 06.03.2009
(51) Int. Cl.: A61B 10/02

(54) **Eye sampling device**
Augenprobenvorrichtung
Dispositif d'échantillonnage d'ýil

(43) Date of publication of application: 08.09.2010
(73) Proprietor: Baudouin, Christophe, 75016 Paris (FR); Elena, Pierre-Paul, 06200 Nice (FR); Groux, Herve, 06560 Le Rouret (FR); Roy, Pierre, 75019 Paris (FR)
(72) Inventor: Roy, Pierre, 75019 Paris (FR); Elena, Pierre-Paul, 06200 Nice (FR); Groux, Hervé, 06560 Le Rouret (FR); Baudouin, Christophe, 75016 Paris (FR)
(74) Representative: Le Forestier, Eric

(56) References cited:
- US-A- 5 135 005
- US-A- 5 137 030
- US-A- 5 259 391
- US-A1- 2001 026 942

## Description

The invention relates to a device with which conjunctival impressions may be taken.

The conjunctival impression technique was developed at the end of the 1970s and is now an accepted technique allowing conjunctival epithelial cells to be collected from the conjunctival mucosa which is an immunologically reactive and complex tissue, in a quasi-painless and non-invasive way, for purposes of analyses and diagnose. The most superficial cells of the conjunctival epithelium, i.e. the cells undergoing desquamation, are collected by this technique. Conjunctival epithelium renews itself from the basal layers, and the most superficial cells which have reached their final differentiation are regularly discharged into the lachrymal fluid. It is this surface layer comprising the terminally differentiated conjunctival cells, which may be sampled by the conjunctival impression for subsequent analysis.

There are three types of cell populations which may be found on conjunctival impressions:
- epithelial covering cells which apart from their participation in coating the conjunctival mucosa, are involved in many biological reactions, particularly in inflammatory and apoptotic processes;
- goblet cells (mucus-secreting cells) which release soluble mucins in the tear film, and which play major defense and regulatory roles at in the ocular surface ; and,
- inflammatory cells including dendritic cells known for their property of immunocompetence, Langerhans cells and also lymphocyte populations localized in the epithelium.

With conjunctival impressions, various cell populations, their size, number, density and pathological modifications may be analyzed and important information may be provided on the status of the eye surface. They also provide information on the conjunctival attack, notably in the field of the dry eye, iatrogenic pathologies induced by collyria, pathologies of the limbus and chronic inflammation of the eye surface.

In order to take a conjunctival impression, the most current present method consists in using membrane filters commonly used in biochemical industry, the most often in cellulose ester (a mixture of cellulose ester and cellulose nitrate) or in polyethersulfone, for example, and which are applied on the conjunctiva, generally without any local anesthesia (See Margarita Calonge et al., Impression cytology of the ocular surface: a review, Experimental Eye Research, 78 (2004), p 457-472). The sampling is totally bloodless, painless and non-invasive contrary to other more aggressive systems such as brushing or biopsy. During the sampling, the filter is generally applied on the upper bulbar conjunctiva of the eye, protected by the eyelid and removed after a contact of 3-5 seconds. The desquamating conjunctival cells then adhere to the membrane surface and form a thin and homogenous cell layer. The filters are then treated in order to recover the cells to be analyzed.

Document US 5 137 030 discloses another method and device for taking a conjunctival impression wherein the device comprises a handle portion and a body portion, which is slotted for scraping conjuctival cells into the slots.

Within the scope of scientific evaluations or when the examinations will be repeated, the impression should always be taken at the same location because there are disparities in the distribution of the sampled cells on the bulbar conjunctiva. This is neither obvious nor easy with the present sampling methods which remain relatively manual even today, in spite of various attempts to improve the procedure (different materials, porosities, holding devices...etc.). They are relatively cumbersome and time-consuming for a clinician or a pathologist within the scope of routine daily use.

An object of the invention is to provide a device for taking an eye impression which is simple to implement while supplying reliable and reproducible sampled impressions.

For this purpose, provision is made according to the invention for, an eye sampling device comprising supporting means comprising a sampling surface, the supporting means being elastically deformable between a rest position and a sampling position and conformed so that, upon being used for sampling, the sampling surface applied in the sampling position in a coinciding way onto a sampling area (2;402;502), is detached therefrom according to a peeling movement when passing from the sampling position to a rest position.

Thus, by the use of elastically deformable supporting means bearing the sampling surface and conformed in order to allow the latter to be detached from the eye sampling area according to a peeling movement, eye impressions which are of optimum quality, may be obtained reliably and reproducibly. Further, this allows a sampling device to be produced, which is simple to use.

Advantageously, but optionally, the device according to the invention has at least one of the following features:
- the sampling surface, in the rest position, includes at least one high point and at least one low point, both distinct so that during the peeling movement, a detachment line gradually passes from the high point to the low point;
- the supporting means are conformed so that, upon being used for sampling, the sampling surface is applied onto the sampling area, according to a movement opposite to the peeling movement when passing from a rest position to a sampling position;
- the supporting means include a block of elastomeric material or of foam;
- the supporting means include a flexible blade;
- the supporting means include a series of spring blades;
- the sampling surface is substantially planar;
- the sampling surface has a section of generally cross-sectional convex shape;
- the sampling surface includes a sampling support and attachment means on the supporting means;
- the attachment means comprise an edge including an extending protrusion which juts out, intended to cooperate with a notch of supporting means of substantially complementary shape;
- the device further includes a body and a piston forming component intended to be slidably moved in the body against elastic return means, the supporting means being attached on one end of the piston forming component;
- the body includes means for separating the sampling surface from the supporting means; and,
- the separation means include shoulders (33,34) intended to bear upon the attachment means (13,14) in order to disengage them.

Other features and advantages of the invention will emerge from the description hereinafter of a preferred embodiment and also of variants. In the appended drawings:
- Figs. 1a and 1b are schematic views of the sampling kinematics achieved by a device according to the invention;
- Figs. 2a and 2b are schematic views of alternative embodiments of the deformable portion of the device according to the invention;
- Figs. 3a-3c are schematic views of alternative embodiments of the sampling surface of the device according to the invention;
- Fig. 4 is a half-sectional view of an embodiment of the device according to the invention;
- Fig. 5 is a simplified cross-sectional view of the kinematics for recovering the eye impression taken by the device of Fig. 4;
- Fig. 6 is a perspective schematic view illustrating the shape of the impression taken by the device of Fig. 4;
- Figs. 7a and 7b are three-dimensional schematic views of alternative shapes of impressions taken by a device according to the invention; and,
- Fig. 8 is an anatomical view of an eye intended to receive the device according to the invention.

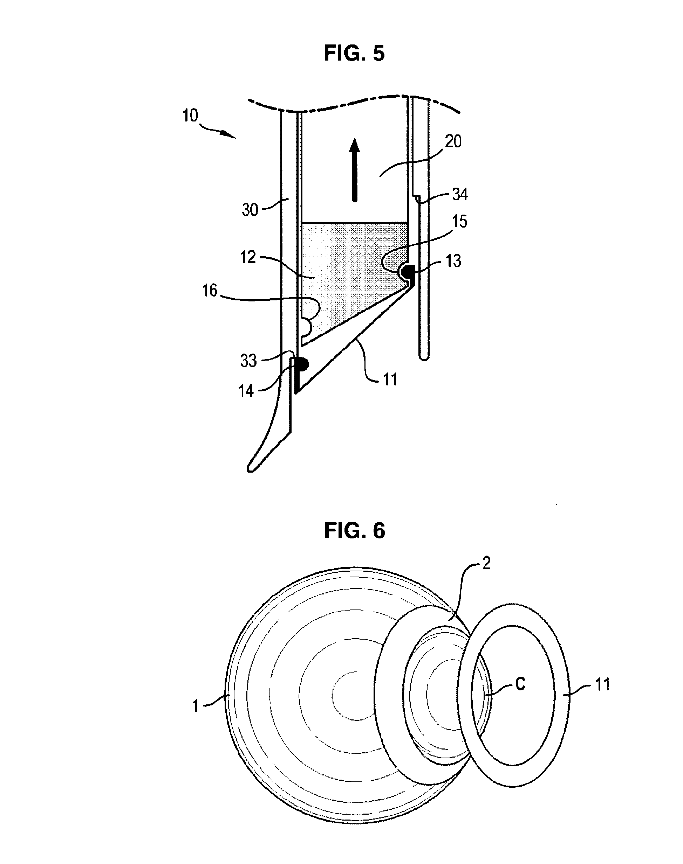

As a liminary remark, the sampling area 2 of an eye impression is located on the conjunctiva, an area located at the periphery of the cornea, which extends from the limbus which delimits the junction between the cornea (nominal diameter of the cornea: about 12 mm for an adult) and the sclera, and extends outwardly to an area externally delimited by a circle with a diameter of about 22 mm and centered on the cornea. For example, as illustrated in Fig. 6, the sampling area 2 (and therefore the sampling surface 11) is ring-shaped with an inner diameter of about 12 mm and an outer diameter of about 17 mm. For a child, whose eye has not reached the adult size, these dimensions have to be adapted in proportion.

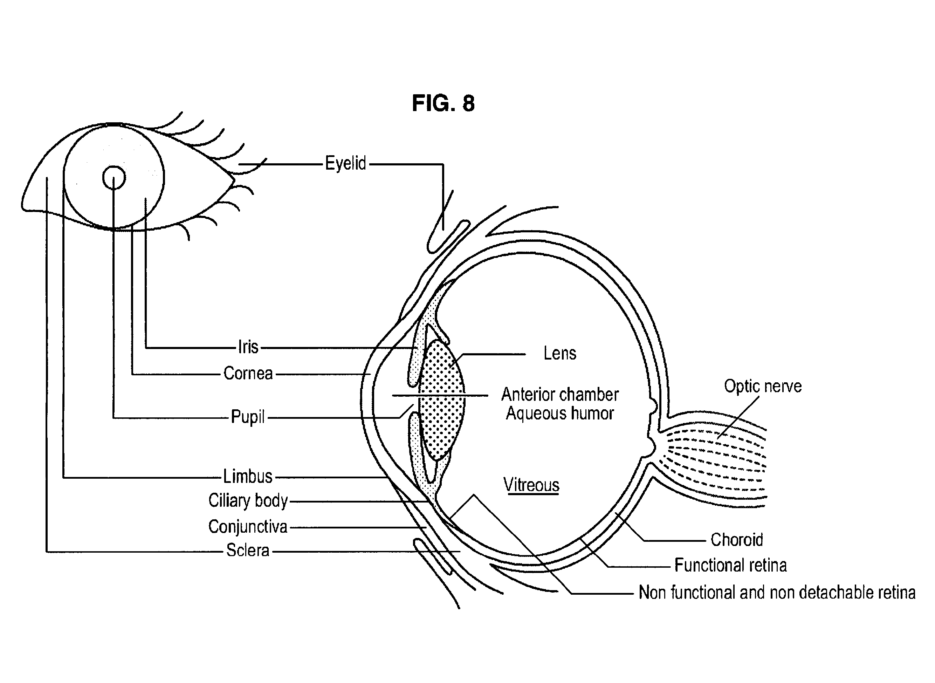

The area 2 for taking an eye impression is moreover located on the eyeball which may be assimilated to a sphere with a diameter of 24 mm +/- 1 mm, except at the cornea. The eye's anatomy is recalled and illustrated in Fig. 8.

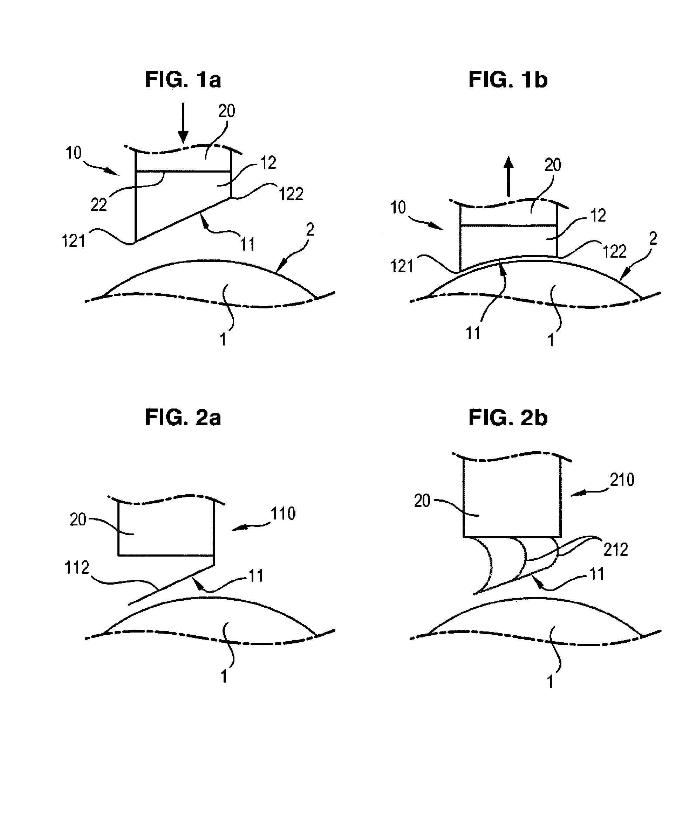

With reference to Figs. 1a and 1b, we shall describe the sampling kinematics of a device 10 for taking an eye impression according to the invention.

This kinematics is important in order to ensure reliability and reproducibility as well as quality of the eye impressions taken by means of the device 10 according to the invention in order to maximize the quality of the sampled cells while maintaining their integrity.

As a matter of principle, the device 10 according to the invention includes a piston forming component 30 having an end 22 on which supporting means 12 are attached. These supporting means 12 are elastically deformable between a rest position illustrated in Fig. 1a and a sampling position illustrated in Fig. 1b. On the other hand, these supporting means include a sampling face or surface 11 which is intended to face and to be pressed against a sampling area 2 of an eye 1. This sampling surface is made in one or more suitable materials forming a support for taking eye impressions and moreover known to one skilled in the art. Finally, the sampling surface, when the supporting means 12 are in the rest position, has at least one low point 121 (or low contact line) and at least one high point 122 (or high contact line), along a longitudinal axis of the device depending on the direction in which a sampling movement is performed.

This sampling movement is obtained by presenting the sampling surface 11 in a non-parallel or non-coinciding way towards the sampling area 2 of the eye surface. Thus, the sampling surface 11, defined by a planar geometry here, is presented so as to face the sampling area 2 of the eye surface according to a given orientation, so that, at the time of contact between both surfaces, there is no coincidence of these surfaces. Indeed, contact between the sampling surface 11 and the sampling area 2 of the eye 1 is initiated by the contact of the low point (or low line) 121 on said sampling area and proceed by moving this contact point (or line) along the sampling surface 11, by sweeping through the latter, right up to the high point (or high line) 122 which lastly comes into contact with the sampling area 2, and this under the effect of a movement along the longitudinal axis of the device 10 according to the invention as illustrated by the arrow of Fig. la. As the contact of the sampling surface 11 gradually advances on the sampling area 2 of the eye surface, the sampling surface 11 gradually deforms so as to become coincident with the sampling area 2 of the eye surface. This deformation also affects the supporting means 12 which deform elastically, until the sampling surface 11 is « stuck » on, i.e. intimately contacting, the sampling area 2 of the eye 1 as this is illustrated in Fig. 1b. There is then coincidence in all points between the sampling surface 11 and the sampling area 2.

The sampling support, once it is placed on the eye 1, is then detached from the eye 1 in the least traumatic way, by exerting a peeling movement. Indeed, with a stress mode of the peeling type, concentrated stresses may exist at the location of the detachment which, here, is a point on the schematic view of Figs. 1a and 1b (or a line passing through this point) and which, during detachment, passes from the high point (or high line) 122 to the low point (or low line) 121, while the sampling surface 11 under the effect of the supporting means 12 which gradually resume their initial resting shape under the effect of the elastic forces, initial shape which does not coincide with the sampling area 2 of the eye 1.

The supporting means 12 are made in a block of elastically deformable material such as a foam or a suitable elastomer (elastic polymer): polyurethane, polyethylene, silicone foam, a thermoplastic elastomer such as Styrene-ethylene/butylene-styrene (SEBS), Styrene-ethylene/propylene-styrene (SEPS) ethylene-propylene copolymer/polypropylene (EPDM-PP), polyether block amides (PEBAX), polyurethane or further a vulcanizable elastomers such as silicone rubber, latex, polybutadiene, a fluorinated elastomer, polychloroprene, polyisoprene...etc.

Two alternative embodiments of the supporting means are illustrated in Figs. 2a and 2b. In Fig. 2a, the supporting means are produced as a flexible blade 112 mounted on the component 20 forming a piston via a supporting rod. In Fig. 2b, the supporting means are produced as a plate supported by spring blades 212, for example made in plastic, in metal or any other material having similar elastic properties.

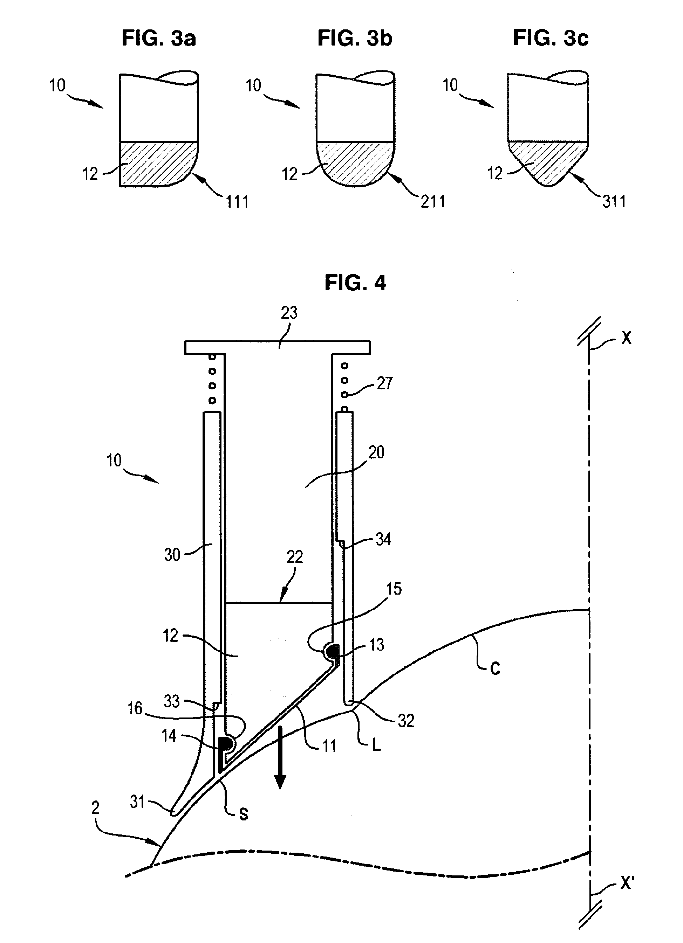

Likewise, with reference to Figs. 3a-3c, the sampling surface alternatively has different convex sectional geometries:
- as a quarter of a circle or an ellipse (Fig. 3a)
- as a half-circle or half-ellipse (Fig. 3b)
- as a substantially triangular or conical tip (Fig. 3c) which respectively corresponds to volume portions of spheres or ellipsoids.

For these different geometries, the sampling surface 111,211,311 always has a low point and at least one high point in order to be able to allow the sampling movement according to the kinematics described earlier, with reference to Figs. 1a and 1b. Thus, regardless of the geometry, the contact between the sampling surface 11,111,211,311 and the sampling area 2 of the eye surface is gradually made and as a dynamic contact point or/and then line gradually sweeping through the sampling area right up to intimate contact between both surfaces, the sampling surface being deformed as well as the supporting means.

Upon removing or detaching the sampling surface, the progressive movement of this same dynamic contact line is the inverse of the movement performed during the contacting.

Thus, the detachment movement is analogous to a peeling movement with which the cells of the sampling area 2 may be gradually detached from the eye surface, which have then adhered onto the sampling support of the sampling surface. This adhesion on the sampling support is achieved according to different well-known principles: by hydrophilicity, by standard surface tension, by adhesion of the cell mucus, or anionic electrostatic charges of the cells.

With reference to Fig. 4, we shall describe an embodiment of a device 10 for taking eye impressions according to the invention which applies the principles and characteristics described earlier. The device 10 here is ring-shaped with a longitudinal axis X-X. It includes a piston 20. The piston 20 includes an upper end 23 comprising an interface for applying said piston 20. It includes an opposite end 22 substantially perpendicular to the longitudinal axis X-X on which the supporting means 12 are positioned and attached, here as a sectional shape of a elastomeric trapezoidal block (or of a block formed with a truncated cylinder, an upper end of which is perpendicular to the axis, in contact on the end 22 of the piston 20 and an opposite lower face tilted with respect to said axis). These supporting means 12 as seen earlier, are elastically deformable between a rest position and a sampling position. An end of the supporting means 12, tilted with respect to the longitudinal axis, bears the sampling surface 11.

This sampling surface 11 includes the sampling support intended to collect an eye impression upon sampling on a sampling area 2 of the eye 1. The sampling support includes at the periphery, around the sampling surface 11, a flexible edge 13, 14 (added by insert-molding, adhesive bonding, welding...etc.), this edge having a protrusion directed radially towards the supporting means 12, which will fit onto a notch 15, 16 located on the perimeter of the supporting means 12. Once it is in place, the edge 13, 14 extends and protrudes from the supporting means 12.

In the case illustrated in Fig. 4, as the device 10 according to the invention is ring-shaped, the sampling support is itself also ring-shaped and includes a first edge 13 on a radially inner periphery and a second edge 14 on a radially outer periphery. Also, the supporting means 12 are ring-shaped and include a first notch 15 on a radially inner face into which the protrusion of the first edge 13 will fit and a second notch 16 on a radially outer face into which the protrusion of the second edge 14 will fit.

The piston 20 is slidably mounted in a body 30 of the device 10 according to the invention and this against an elastic return means 21, here as a compression spring. The body 30 includes a first radially inner wall including an end 32 intended to come into contact with the eye 1 during sampling. Additionally, the radially inner wall of the body 30 includes a radially outer shoulder 34, optionally with a frusto-conical shape, the role of which will be described subsequently with reference to Fig. 5.

Given that the conjunctiva is a very mobile tissue relatively to the underlying sclera S, that the eye 1 is capable of reflex eye movements upon sampling (with the risk of damaging the sample by a shearing effect between the sampling support placed on the conjunctiva and the conjunctiva), that the conjunctiva is attached at the limbus L and since there is a change in the radius of curvature between the cornea C and the sclera S, the end 32 of the device 10 according to the invention includes a semi-circular supporting area intended to come into contact, by fitting its contour, with the limbus L of the eye 1, thereby ensuring proper positioning on the one hand and properly maintaining the relative position between the eye and the device according to the invention during the sampling, on the other hand.

The body 30 further includes a second radially outer wall comprising an end 31 intended to come into contact with the eye 1 during sampling. Additionally, the radially outer wall of the body 30 includes a radially inner shoulder 33, the role of which will be described subsequently with reference to Fig. 5.

Given that the edges of the eyelids are often contaminated by bacterial strains different from those present at the eye surface, there is a risk of contamination of the taken sample specifically dedicated to the analysis of this surface. Consequently, before putting the sampling support in contact with the sampling area of the eye, the surface including the sampling area of the edge of the eyelids has to be isolated. This is the role of the end 31 which is conformed in order to face the eyelid, by being accommodated below the eyelid, while maintaining the latter open and preventing any crossed contamination between the sample and the eyelid. For this, the end 31 has a foot-shaped section, the sole of which has a section of a concave shape mating that of the eye surface upon which the end 31 is intended to bear during sampling, the eyelid will then come into contact and be retained by the top of said foot.

On the other hand, it should be noted that the eye surface is covered with a lachrymal film consisting of tears and of a thin layer of lipids secreted by the Meibomian glands located at the lid margin of the lower and upper eyelids. This thin lipidic and hydrophobic layer will limit or prevent adhesion of the cells on the highly hydrophilic surface of the sampling support. Consequently, before having the sampling support come into contact with the sampling area of the eye, the tear film has to be broken. This is the additional role of the ends 31 and 32 of the body 30. For this, these ends 31 and 32 are made in or covered with a semi-rigid or elastomeric (so as not to injure the eye surface and to achieve intimate contact with the latter) material and which is both absorbent and lipophilic so as to absorb the lipid portion of the tear film at the sampling area of the sampling surface thereby delimited by these ends 31 and 32.

With reference to Fig. 5, we shall describe operation of the device 10 for taking an eye impression according to the invention which has just been described.

The device 10 according to the invention is placed on the eye 1 so that the end 32 of the body 30 will contact the limbus L and that the end 31 of the body 31 is inserted under the eyelids. Next, pressure is exerted on the end 23 of piston 20 in order to accomplish the sampling. This pressure is exerted along the longitudinal axis X-X and oriented towards the eye 1 (see the arrow of Fig. 4). The sampling support 11 is then pressed on the eye according to the sampling kinematics which was described earlier with reference to Figs. 1a and 1b. After a few seconds, the pressure on the end 23 of the piston 20 is released, the so compressed springs 24 exert a return force on the piston 20 forcing it to slide in the body 30 along the longitudinal axis X-X in the direction of the arrow as illustrated in Fig. 6. The sampling support 11 is driven by the piston and is delicately detached according to the peeling movement described earlier. There is no suction cup effect between the sampling support 11 and sampling area 2 of the eye. The device 10 is then removed from the eye.

Now the sampling support 11 remains to be recovered in order to be able to carry out the intended analyses. Recovery of the sampling support 11 is performed after having placed at least the portion including the sampling support 11 of the device according to the invention inside a collecting means, such as for example a collecting tube.

The piston 20 is then pulled backwards according to the arrow of Fig. 6 and the edges 14 and 13 of the sampling support 11 will bear upon the shoulders 33 and 34 of the body 30. As the movement of the piston 20 being pulled backwards continues, the edges 14 and 13 retained by the shoulders 33 and 34, each protrusion of the edges 14 and 13 are then disengaged from the notches 16 and 15 of the supporting means 12, respectively. The sampling support 11 is detached and falls into the collecting means.

Alternatively, the detachment of the sampling support 11 is carried out according to a gradual movement defined by the initial position of the shoulders 33 and 34 along the travel covered by the piston 20 when it is pulled backwards in the body 30: the edge 14 will first bear upon the shoulder 33 and its protrusion disengages from the notch 16, and then the edge 14 will first bear upon the shoulder 34 and its protrusion disengages from the notch 15, thereby releasing the sampling support 11. For this, the shoulders 33 and 34 are not located at a same height along the axis of the device and the edges 14 and 13 themselves are not at the same height, which is the case in the example of Fig. 5. Alternatively, the shoulders 33 and 34 are located at a same height along the axis of the device and the edges 14 and 13 themselves are not at the same height. In still another alternative, the shoulders 33 and 34 are not located at a same height along the axis of the device and the edges 14 and 13 themselves are at the same height.

The sampling support 11 falls into the collecting means without any other handling from behalf of the user and with very limited contact with open air. This thereby reduces the risks of accidental contamination (desirable for analyzing the germs present at the eye surface and detecting infestations of microorganisms, for example) and of damage before utilization. Indeed, cytology for example requires the cells to be very well attached onto the sampling support 11 so as to be marked with fluorescent markers and allow observation by conventional microscopy (with transparization of the support) or confocal microscopy.

Cytometry as for it, requires the cells to adhere as much as possible on the support, while allowing to be easily detached when desirable into a collecting solution with order to proceed to cell counting and sorting.

Finally, sample analysis by means of polymerase chain reaction (PCR), assumes destruction of the sampling support into the products used for cell lysis allowing DNA extraction.

By using the device according to the invention, eye impressions may be optimized to the various kinds of analysis techniques by using different supports adapted to the analysis procedure.

The sampling support 11 is here an insert-molded membrane filter type with a flexible plastic edge (which has the advantage of being a versatile solution, allowing the use of several types of membranes). Alternatively, it is possible to mould a shaped one-piece part (membrane + edge) in a plastic material ad *hoc,* and if necessary including a treated surface, optimized for the sampling (roughness, plasma treatment in order to increase hydrophilicity,...etc.) in order to produce the sampling support.

Considering the foregoing, the device 10 according to the invention therefore provides control of the two following parameters when placing and removing the sampling support, for making the samplings more reliable:
- Movement for placing the support
- Pressure (controlled and reproducible)

Further, with the device 10 according to the invention, as described earlier, the problem of the tear film may be solved, and the sampling surface may be defined and localized always at the same location and the eye may be immobilized, as well as recovery may be optimum without damaging the taken eye impression.

As alternative embodiments, the device according to the invention, which has just been described according to one embodiment, where it has an axisymmetrical ring shape, may appear in any geometrical (sector, round, oval, ovoid ...etc.) shape able to be inscribed between the diameter at the limbus and the diameter of about 22 mm.

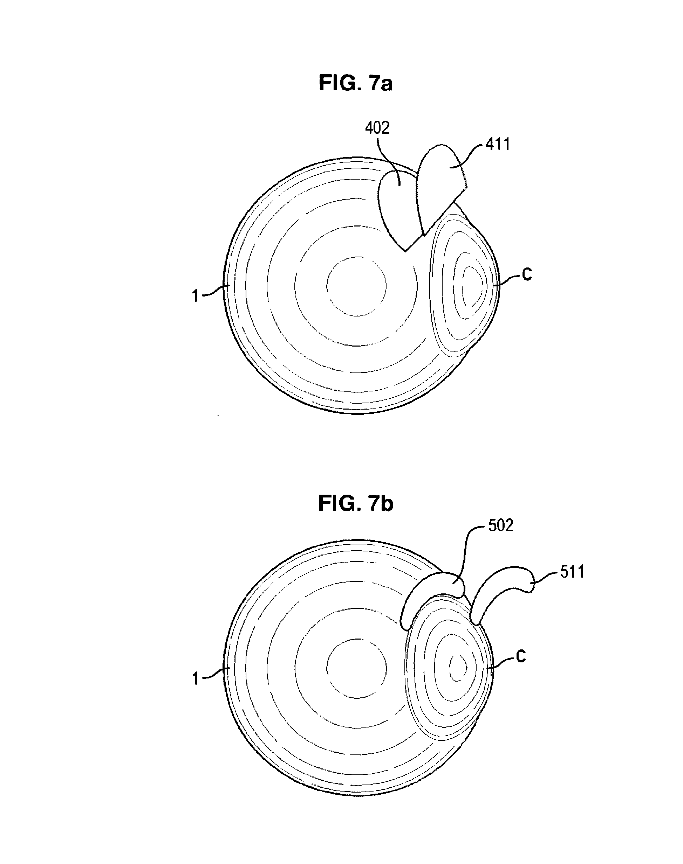

A first alternative embodiment illustrated in Fig. 7a delimits a sampling area 402 (and so the sampling support 411) formed by a half-disc with a diameter of about 13 mm, the centre of which is tangential at the limbus and placed on the upper area of the conjunctiva. The disc is inscribed in the ring-shaped sampling surface with an inner diameter of about 12 mm and an outer diameter of about 18.5 mm in this case.

A second alternative embodiment illustrated in Fig. 7b delimits a sampling area 502 (and so the sampling support 511) as a sector with an inner diameter of about 12 mm at the limbus and an outer diameter of about 17 mm.

In both of these alternative embodiments, as well as in others where the device according to the invention does not have an axisymmetrical ring shape, the shoulders 33 and 34 form a single and same shoulder inside the body of the device, as well as the notches 13 and 14 which are only a single notch at the periphery of the supporting means 12.

The device according to the invention described earlier therefore operates by having the conjunctiva come into contact with a material allowing controlled adhesion of the cells. Most particularly, it ensures that:
(i) the parameters which may influence the quality of the taken sample (amount of non-lysed cells) are stabilized such as pressure, application time and sampling kinematics.

Additionally with it, it is possible to:
(ii) accurately delimit a determined eye surface located on the pars plana;
(iii) immobilize the eye during the sampling time;
(iv) isolate the sample from the tear film and from the lid margins; and,
(v) prevent contamination of the sample (no contact with the eyelids, the physician and the environment).

And this while ensuring that it is as atraumatic as possible.

The material allowing the sampling and forming the sampling support 11 (foam, woven or non-woven fabric filter, hydrogel, polymer whether treated or not...etc.) is compatible with the present methods of analysis, i.e:
- Conventional microscopy (with transparization of the support) or confocal microscopy (without transparization) for cytology;
- Flow cytometry (detachment of the cells from the support);
- PCR assays (either detachment of the cells from the support, or complete dissolution of the filter in the lysis solution of the cells).

Other exemplary uses of the taken sample may relate to recovery of DNA (within the scope of forensic medicine for example) or to the analysis of germs present at the eye surface (viruses and bacteria).

It is also possible to search for atmospheric pollutants or even radioactive particles in the sample and it would thus be simpler to measure contamination than by means of a blood sample. When the eye is open, it is continually in contact with its environment and exposed to airborne substances or to substances present in the atmosphere, and in some way, by collecting these substances, it is acting as a sensor. Collection of substances collected by the eye with this method may have advantages as compared with other techniques used such as skin biopsies or blood samples. More generally, it is/will be possible to collect and also analyze the composition of tears and to detect the content of chemical mediators, hormones (insulin,...), DNA, blood proteins, ions, enzymes, in normal and/or pathological situations.

In view of the teaching contained herein, one skilled in the art can identify various other configurations to the invention without departing from the scope thereof.

## Claims

1. An eye sampling device (10) comprising supporting means (12;112;212) comprising a sampling surface (11;111;211;311;411;511), **characterized in that** the supporting means are elastically deformable between a rest position and a sampling position and conformed so that, upon being used for sampling, the sampling surface applied in the sampling position in a coinciding way onto a sampling area (2;402;502), is detached therefrom according to a peeling movement when passing from the sampling position to a rest position.

2. The device according to claim 1, **characterized in that** the sampling surface, in the rest position, includes at least one high point (122) and at least one low point (121), both distinct so that during the peeling movement, a detachment line gradually passes from the high point (122) to the low point (121).

3. The device according to claim 1 or 2, **characterized in that** the supporting means are conformed so that, upon being used for sampling, the sampling surface is applied onto the sampling area, according to a movement opposite to the peeling movement when passing from a rest position to a sampling position.

4. The device according to any of claims 1 to 3, **characterized in that** the supporting means include a block of elastomeric material or of foam (12).

5. The device according to any of claims 1 to 3, **characterized in that** the supporting means include a flexible blade (112).

6. The device according to any of claims 1 to 3, **characterized in that** the supporting means include a series of spring blades (212).

7. The device according to any of claims 1 to 6, **characterized in that** the sampling surface is substantially planar (11).

8. The device according to any of claims 1 to 6, **characterized in that** the sampling surface has a section (111;211;311) of generally cross-sectional convex shape.

9. The device according to any of claims 1 to 8, **characterized in that** the sampling surface includes a sampling support and attachment means (13,14) on the supporting means.

10. The device according to claim 9, **characterized in that** the attachment means comprise an edge (13,14) including an extending protrusion which juts out, intended to cooperate with a notch (15,16) of supporting means of substantially complementary shape.

11. The device according to any of claims 1 to 10, **characterized in that** it further includes a body (30) and a piston forming component (20) intended to be slidably moved in the body against elastic return means (24), the supporting means being attached on one end (22) of the piston forming component.

12. The device according to claim 11, **characterized in that** the body includes means (33,34) for separating the sampling surface from the supporting means.

13. The device according to claim 12, **characterized in that** the separation means include shoulders (33,34) intended to bear upon the attachment means (13,14) in order to disengage them.

## Patentansprüche

1. Augen-Probenahmevorrichtung (10), die eine Halteeinrichtung (12; 112; 212) mit einer Probenahmefläche (11; 111; 211; 311; 411; 511) aufweist, **dadurch gekennzeichnet, dass** die Halteeinrichtung zwischen einer Ruheposition und einer Probenahmeposition elastisch verformbar und so angepasst ist, dass bei Gebrauch zur Probenahme die in der Probenahmeposition koinzidierend auf ein Probenahmegebiet (2; 402, 502) aufgesetzte Probenahmefläche von diesem in Übereinstimmung mit einer Schälbewegung abgelöst wird, wenn sie aus der Probenahmeposition in eine Ruheposition übergeht.

2. Vorrichtung nach Anspruch 1, **dadurch gekennzeichnet, dass** die Probenahmefläche in der Ruheposition mindestens einen Hochpunkt (122) und mindestens einen Tiefpunkt (121) aufweist, die beide so verschieden sind, dass im Verlauf der Schälbewegung eine Ablöselinie vom Hochpunkt (122) zum Tiefpunkt (121) allmählich übergeht.

3. Vorrichtung nach Anspruch 1 oder 2, **dadurch gekennzeichnet, dass** die Halteeinrichtung so angepasst ist, dass bei Gebrauch zur Probenahme die Probenahmefläche auf das Probenahmegebiet in Übereinstimmung mit einer zur Schälbewegung entgegengesetzten Bewegung aufgesetzt wird, wenn sie aus einer Ruheposition in eine Probenahmeposition übergeht.

4. Vorrichtung nach einem der Ansprüche 1 bis 3, **dadurch gekennzeichnet, dass** die Halteeinrichtung einen Block aus Elastomermaterial oder aus Schaumstoff (12) aufweist.

5. Vorrichtung nach einem der Ansprüche 1 bis 3, **dadurch gekennzeichnet, dass** die Halteeinrichtung eine flexible Klinge (112) aufweist.

6. Vorrichtung nach einem der Ansprüche 1 bis 3, **dadurch gekennzeichnet, dass** die Halteeinrichtung eine Folge von Federklingen (212) aufweist.

7. Vorrichtung nach einem der Ansprüche 1 bis 6, **dadurch gekennzeichnet, dass** die Probenahmefläche im Wesentlichen eben (11) ist.

8. Vorrichtung nach einem der Ansprüche 1 bis 6, **dadurch gekennzeichnet, dass** die Probenahmefläche einen Schnitt (111; 211; 311) mit allgemein konvexer Querschnittform hat.

9. Vorrichtung nach einem der Ansprüche 1 bis 8, **dadurch gekennzeichnet, dass** die Probenahmefläche eine Probenahmestütz- und - befestigungseinrichtung (13, 14) auf der Halteeinrichtung aufweist.

10. Vorrichtung nach Anspruch 9, **dadurch gekennzeichnet, dass** die Befestigungseinrichtung eine Kante (13, 14) mit einem ausgezogenen Vorsprung aufweist, der herausragt und mit einer Kerbe (15, 16) der Halteeinrichtung mit im Wesentlichen komplementärer Form zusammenwirken soll.

11. Vorrichtung nach einem der Ansprüche 1 bis 10, **dadurch gekennzeichnet, dass** sie ferner einen Grundkörper (30) und eine Kolbenbildungskomponente (20) aufweist, die im Grundkörper gegen eine elastische Rückführeinrichtung (24) gleitfähig bewegt werden soll, wobei die Halteeinrichtung an einem Ende (22) der Kolbenbildungskomponente befestigt ist.

12. Vorrichtung nach Anspruch 11, **dadurch gekennzeichnet, dass** der Grundkörper eine Einrichtung (33, 34) zum Trennen der Probenahmefläche von der Halteeinrichtung aufweist.

13. Vorrichtung nach Anspruch 12, **dadurch gekennzeichnet, dass** die Trenneinrichtung Schultern (33, 34) aufweist, die an der Befestigungseinrichtung (13, 14) anliegen sollen, um sie abzulösen.

## Revendications

1. Dispositif d'échantillonnage oculaire (10) comprenant des moyens de support (12 ; 112 ; 212) comprenant une surface d'échantillonnage (11 ; 111 ; 211 ; 311 ; 411 ; 511), **caractérisé en ce que** les moyens de support sont élastiquement déformables entre une position de repos et une position d'échantillonnage et conformés de telle sorte que, lorsqu'ils sont utilisés pour l'échantillonnage, la surface d'échantillonnage appliquée dans la position d'échantillonnage d'une manière coïncidente sur une zone d'échantillonnage (2 ; 402 ; 502), est détachée de celle-ci selon un mouvement de décollement lors du passage de la position d'échantillonnage à une position de repos.

2. Dispositif selon la revendication 1, **caractérisé en ce que** la surface d'échantillonnage, dans la position de repos, comprend au moins un point élevé (122) et au moins un point bas (121), tous deux distincts de telle sorte que lors du mouvement de décollement, une ligne de détachement passe progressivement du point élevé (122) au point bas (121).

3. Dispositif selon la revendication 1 ou 2, **caractérisé en ce que** les moyens de support sont conformés de telle sorte que, lorsqu'ils sont utilisés pour l'échantillonnage, la surface d'échantillonnage est appliquée sur la zone d'échantillonnage, selon un mouvement opposé au mouvement de décollement lors du passage d'une position de repos à une position d'échantillonnage.

4. Dispositif selon l'une quelconque des revendications 1 à 3, **caractérisé en ce que** les moyens de support comprennent un bloc de matière élastomère ou de mousse (12).

5. Dispositif selon l'une quelconque des revendications 1 à 3, **caractérisé en ce que** les moyens de support comprennent une lame flexible (112).

6. Dispositif selon l'une quelconque des revendications 1 à 3, **caractérisé en ce que** les moyens de support comprennent une série de lames ressorts (212).

7. Dispositif selon l'une quelconque des revendications 1 à 6, **caractérisé en ce que** la surface d'échantillonnage est sensiblement plane (11).

8. Dispositif selon l'une quelconque des revendications 1 à 6, **caractérisé en ce que** la surface d'échantillonnage comprend une section (111 ; 211 ; 311) de forme convexe généralement transversale.

9. Dispositif selon l'une quelconque des revendications 1 à 8, **caractérisé en ce que** la surface d'échantillonnage comprend un support d'échantillonnage et des moyens de fixation (13, 14) sur les moyens de support.

10. Dispositif selon la revendication 9, **caractérisé en ce que** les moyens de fixation comprennent un bord (13, 14) comprenant une protubérance allongée en saillie, conçue pour coopérer avec une encoche (15, 16) des moyens de support de forme sensiblement complémentaire.

11. Dispositif selon l'une quelconque des revendications 1 à 10, **caractérisé en ce qu'**il comprend en outre un corps (30) et un composant formant piston (20) conçu pour être déplacé de manière coulissante dans le corps contre les moyens de retour élastiques (24), les moyens de support étant fixés sur une extrémité (22) du composant formant piston.

12. Dispositif selon la revendication 11, **caractérisé en ce que** le corps comprend des moyens (33, 34) pour séparer la surface d'échantillonnage des moyens de support.

13. Dispositif selon la revendication 12, **caractérisé en ce que** les moyens de séparation comprennent des épaulements (33, 34) conçus pour appuyer sur les moyens de fixation (13, 14) afin de les libérer.
